# FASCICULE DE BREVET EUROPEEN

(11) **EP 2 049 074 B1**
(45) Date de publication et mention de la délivrance du brevet: **28.07.2010**
(21) Numéro de dépôt: 07787699.3
(22) Date de dépôt: 18.07.2007
(51) Int. Cl.: A61K 8/64, A61Q 17/04, A61Q 19/04, A61Q 5/10

(54) **Utilisation non-thérapeutique d'un hydrolysat de protéines de riz en tant que principe actif pigmentant**
Nicht-therapeutische Verwendung eines Reisprotein-Hydrolysats als pigmentierender Wirkstoff
Non-therapeutic use of a rice protein hydrolysate as pigmenting active principle

(30) Priorité: 18.07.2006 FR 0606780
(43) Date de publication de la demande: 22.04.2009
(73) Titulaire: Laboratoires Expanscience, 92400 Courbevoie (FR)
(72) Inventeur: MSIKA, Philippe, F-78000 Versailles (FR); NAAÏMI, Dalale, F-21000 Dijon (FR)
(74) Mandataire: Warcoin, Jacques
(86) Numéro de dépôt international: PCT/EP2007/057441
(87) Numéro de publication internationale: WO 2008/009709

(56) Documents cités:
- EP-A- 0 884 045
- EP-A- 1 656 970
- WO-A-96/22698
- FR-A- 2 553 285
- US-A- 5 662 890

## Description

La présente invention se rapporte à l'utilisation non thérapeutique d'au moins un hydrolysat de protéines de riz, en tant que principe actif pigmentant, dans une composition cosmétique ou nutraceutique.

La mélanine est un pigment responsable, chez l'homme, de la coloration de la peau, des cheveux, des yeux. La mélanine est produite par les mélanocytes, cellules qui se trouvent dans l'épiderme à partir d'un acide aminé : la Tyrosine. La tyrosinase transforme la tyrosine en DOPA puis DOPAquinone. De nombreuses enzymes interviennent ensuite pour former la mélanine.

Dans le mélanocyte, la mélanine se trouve dans des organites intra-cellulaires (les mélanosomes). Ces mélanosomes sont transférés par les mélanocytes grâce à leurs ramifications (dendrites) vers les kératinocytes voisins. Le mélanosome assure sa fonction protectrice vis à vis des UV en se plaçant au dessus du noyau du kératinocyte à la manière d'un parasol.

Les mélanocytes se situent au niveau de la jonction dermo-épidermique et s'insèrent entre les kératinocytes de la couche basale. 1 mélanocyte protège 36 kératinocytes, c'est ce qu'on appelle l'unité mélanique.

Chez l'homme, la mélanine est responsable de la pigmentation constitutive et du bronzage. Via le bronzage, elle protège l'ADN des cellules de la peau des rayons ultraviolets (UV) du soleil, UVA et UVB. Lors d'une exposition prolongée au soleil, le corps bronze car il fabrique plus de mélanine, il est ainsi mieux protégé contre les UV. La mélanine agit en absorbant les radiations ultraviolettes en restituant sous forme de chaleur l'énergie reçue. La définition des phototypes humains intègre la notion de couleur de peau initiale et de capacité à bronzer et rougir au soleil. Il y a donc un intérêt pour un phototype donné d'accéder à un phototype supérieur qui lui conférera une photoprotection plus importante via un taux de pigmentation supérieur.

En raison d'une surexposition solaire et/ou d'antécédents familiaux génétiques ou d'une maladie de peau, des tâches blanches inesthétiques, consistant en une perte de pigmentation de la peau, peuvent apparaître. L'affection de la peau de ce type la plus connue est le vitiligo.

Des tâches blanches peuvent également apparaître sur la peau (de patients bronzés principalement) dans le cas du *Pityriasis versicolor,* affection due à la prolifération excessive d'un champignon appartenant au groupe des levures du genre *Malassezia.* De nos jours, on sait traiter ces levures. Toutefois, le traitement arrête l'infection mais les tâches blanches persistent, normalement jusqu'à une prochaine exposition au soleil. De même, il peut apparaître des hypochromies après des cicatrices post traumatiques ou après brûlures, lors de l'installation de vergetures dites anciennes (vergetures blanches non inflammatoires) et enfin après le traitement des dartres. Pour accélérer la disparition de ces tâches blanches, un traitement favorisant la pigmentation peut être conseillé.

L'apparition de cheveux blancs (canitie) provient d'un arrêt de production de mélanine au niveau de leur bulbe. On ne connaît pas encore très bien le mécanisme de cet arrêt de production. En revanche, il s'agit d'un mécanisme héréditaire et lié à l'âge.

De nos jours, on cherche à avoir une peau bronzée tout en limitant ou annulant les durées d'exposition aux UV (naturels ou non) et en préservant la peau du vieillissement cutané précoce et autres effets secondaires dus aux UV.

Le document FR-A-2 553 285 concerne des compostions cosmétiques colorantes pour la coloration de supports kératiniques tels que les cheveux ou bien destinées à être appliquées sur le revêtement cutané. La substance active est un dérivé protéinique.

US-A-5 662 890 décrit des compostions cosmétiques pour le bronzage artificiel de la peau. Les compositions ne comprennent ni alcool ni huile et sont applicables en spray. EP-A-0 884 045 décrit des formulations comprenant de la dihydroxyacétone pour le bronzage de la peau. Ces compositions ont une stabilité améliorée.

WO 96/22698 décrit des hydrolysats de protéines de riz ainsi qu'un procédé d'obtention de ces hydrolysats.

EP-A-1 656 970 décrit des compositions cosmétiques comprenant des hydrolysats de protéines de riz et un agent augmentant la synthèse des glycosaminoglycanes. Ces compositions ont un effet anti-âge ou anti-vieillissement de la peau.

Aucun de ces documents ne décrit l'utilisation d'un hydrolysat de protéines de riz comme agent actif pour pigmenter, hyperpigmenter ou repigmenter la peau et les phanères.

Les inventeurs ont découvert, de manière surprenante et inattendue, que des hydrolysats de protéines de riz (également appelés peptides de riz) avaient la propriété de stimuler la pigmentation cutanée. L'application de l'actif permet une pigmentation constitutive plus importante et un bronzage plus rapide et plus intense pour un temps d'exposition plus courut. Par conséquent, l'apparition des facteurs du vieillissement cutané et autres désagréments dus aux UV se trouverait ralentie.

L'invention a donc pour premier objet l'utilisation non thérapeutique d'un hydrolysat de protéines de riz en tant que principe actif pigmentant, dans une composition cosmétique, ou nutraceutique pour l'homme et les animaux.

Cette composition est destinée à stimuler la pigmentation cutanée (qui peut être constitutive ou acquise), elle peut donc être utilisée en tant qu'accélérateur de bronzage.

La composition peut être utilisée dans les cabines de bronzage soit dans un but purement esthétique (bronzer) soit dans un but dermatologique (renforcer le phototype de patients ayant une peau claire et sensible au soleil).

Ainsi, selon des variantes avantageuses de l'invention, la composition selon l'invention est destinée à augmenter et intensifier la pigmentation normale de la peau sans soleil. Elle est également destinée à accélérer et intensifier le bronzage. Elle est également destinée à prévenir le photovieillissement.

L'invention a également pour objet une méthode de traitement cosmétique non thérapeutique pour stimuler la pigmentation constitutive ou acquise, caractérisée en ce que l'on applique par voie topique une composition comprenant au moins un hydrolysat de protéines de riz. Cette méthode de traitement cosmétique permet notamment d'accélérer le bronzage et la couleur naturelle de la peau sans soleil ou d'acquérir un bronzage sans ou avec peu de soleil.

Cette composition peut également être utilisée dans le traitement des tâches de dépigmentation (tâches blanches) de la peau, telles que le vitiligo ou les hypopigmentation iatrogènes (dermo-corticoïdes). La composition peut être également utilisée pour repigmenter la peau chez des patients ayant développé unpityriasis, en particulier, un *Pityriasis versicolor.* Elle pourrait aussi permettre aux personnels atteintes de la pathologie dite des enfants de la lune d'avoir une stimulation sans soleil de la pigmentation cutanée.

L'invention a donc aussi pour objet l'utilisation non thérapeutique d'un hydrolysat de protéines de riz pour repigmenter les tâches blanches cutanées. Les tâches de dépigmentation peuvent être dues ou consécutives à une affection ou une sensibilisation choisie dans le groupe constitué par un vitiligo, un pityriasis *(Pityriasis versicolor),* une utilisation de derino-corticoïdes, une cicatrice ou une exposition solaire intensive. Les tâches de dépigmentation peuvent être dues à un photovieillissement important.

L'invention a également pour objet l'utilisation d'un hydrolysat de protéines de riz pour la fabrication d'un soin capillaire pour la prévention et le traitement des cheveux blancs.

Dans le cadre de la présente invention, les expressions « hydrolysats de protéines de riz » et « peptides de riz » désignent toutes deux le produit résultant de l'hydrolyse enzymatique de protéines de riz.

Les peptides de riz sont déjà connus de l'art antérieur et des procédés d'obtention de ces peptides ont déjà été décrits (EP 575 452, WO 02/102347).

Des hydrolysats de protéines de riz sont disponibles dans le commerce auprès de la société SILAB sous la dénomination Nutriskin. Ces hydrolysats comprennent des peptides de faible poids moléculaire (inférieur à 1400 daltons). Ils peuvent être obtenus comme décrit dans la demande internationale WO 02/102347.

Un procédé de préparation d'un hydrolysat de protéines de riz comprend par exemple les étapes consistant à :
- solubiliser des protéines de riz dans l'eau pour obtenir une suspension,
- hydrolyser cette suspension en présence d'une ou plusieurs protéases,
- avantageusement filtrer sur membrane pour éliminer les composés insolubles, l'enzyme résiduelle et les peptides de haut poids moléculaire (supérieur à 10 000 daltons) ;
- et éventuellement, réaliser une filtration stérilisante du concentrat obtenu précédemment.

Les protéines de riz utilisées peuvent être obtenues par broyage des grains de riz (*ex : Oryza sativa*)*.* Des concentrats de protéines de riz sont également disponibles dans le commerce (commercialisés par la société REMY Industries, par exemple).

Avantageusement, le procédé de préparation de l'hydrolysat de protéines de riz ne comprend pas d'étape de fermentation ou de germination. En outre, le riz utilisé dans la composition selon l'invention et/ou mis en oeuvre dans le procédé ci-dessus appartient de préférence au genre *Oryza* et est plus préférentiellement de l'espèce *Oryza sativa* L. Avantageusement, l'hydrolysat de riz utilisé dans la composition selon l'invention, et celui obtenu dans le procédé de préparation ci-dessus, sont issus d'une fraction de riz exempte de son de riz

L'hydrolysat de protéines de riz utilisé dans le procédé de l'invention pourra comprendre des peptides ayant une masse moléculaire moyenne allant de moins de 300 daltons à plus de 10.000 daltons, de préférence une masse moléculaire moyenne allant de 300 daltons à 3500 daltons.

En particulier, on utilisera de préférence un hydrolysat de protéines de riz comprenant des peptides ayant une masse moléculaire inférieure ou égale à 10 000 daltons, ou préférentiellement une masse moléculaire inférieure ou égale à 3 500 Daltons. Selon un mode particulier de l'invention, au moins 75% en masse, avantageusement au moins 80% en masse, plus avantageusement au moins 85% en masse, de peptides ont une masse moléculaire comprise entre 300 et 3 500 Da. En particulier, au moins 50% en masse des peptides, avantageusement au moins 55% en nombre des peptides, ont une masse moléculaire comprise entre 300 et 1 200 Da.

La concentration en peptides de riz dans la composition (cosmétique, dermatologique ou nutraceutique) utilisée selon l'invention est avantageusement comprise entre environ 0,1% et environ 20% en poids, plus avantageusement entre environ 1% et environ 10% en poids, encore plus avantageusement entre environ 4% et environ 6% en poids, par rapport au poids total de la composition.

La composition utilisée selon l'invention peut contenir d'autres actifs à action pigmentante (protection UV en plus) ou colorante de la peau, conduisant à un effet complémentaire ou synergique.

Comme exemple d'agents pigmentants ou colorants, on peut notamment citer
- les agents qui colorent la peau : le dihydroxyacétone, les mélanines ;
- les agents qui stimulent le procédé de pigmentation naturelle : les psolarènes (8-méthoxypsolarène, 5-méthoxypsolarène, 4,5',8-triméthylpsolarène ou des extraits végétaux de *Psorelea corylifolia* et de *Ammi majus*)*,* les caroténoïdes (lycopène, canthaxanthine), les agents stimulant la voie de l'AMP cyclique (1. les analogues de l'AMPc, tels que le 8-bromo-AMPc ou le dibutiryl-AMPc, 2. la forskoline, 3. l'isobutyl-méthyl-xanthine ou la théophylline), les activateurs des protéines kinase C (diacylglycérols, en particulier oléyl-acétyl-glycérol), diols aliphatiques ou cycliques (1,2-propandiol, 5-norbomane-2,2-diméthanol, norbomane-2,2-diméthano), les diols bicycliques monoterpène, les dérivés de tyrosine (L-tyrosine, L-DOPA), le diméthylsulfoxyde, les agents lysomotropiques, les dinucléotides thymidine, les fragments d'ADN, les analogues de l'hormone stimulant les mélanocytes, le 3-isobutyl-1-méthylxanthine, les donneurs d'acide nitrique (David A, Brown, Journal of photochemistry and photobiology B :biology 63 (2001) 148-161) ;
- les extraits végétaux, en particulier les algues, démontrant une activité promélanogène : *Laminaria digitata* (Thalitan de Codif).

La tyrosinase est une enzyme impliquée dans la mélanogénèse. Ainsi, les peptides de riz selon l'invention peuvent être associés avec des actifs capables :
- d'activer les facteurs inducteurs de synthèse de tyrosinase ;
- de promouvoir l'activité enzymatique de la tyrosinase ;
- de promouvoir la glycosilation de la tyrosinase (permet son absorption par les mélanosomes) ;
- d'augmenter l'activité globale des mélanocytes ;
- de promouvoir le transfert de la tyrosinase dans les prémélanosomes ;
- de favoriser les précurseurs de la mélanine ;
- de restreindre l'activité cellulaire, ce qui permet de conserver les kératinocytes chargés en mélanine.

Les peptides de riz peuvent également être associés à des agents oxydants ou anti-oxydants, conduisant à un effet complémentaire ou synergique. Comme exemple d'agents anti-oxydants, on peut notamment citer la vitamine C, la vitamine E, les polyphénols (notamment ceux extraits du thé vert ou de raisin ou de pin), les dérivés soufrés.

Les peptides de riz peuvent également être associés à des protecteurs cellulaires de l'agression solaire, autres que les anti-oxydants, agissant sur les cellules coup de soleil, la p53, les protéines de stress (heat shock proteins), l'apoptose, la lyse des membranes, la libération de cytokines ou de médiateurs cellulaires.

Selon un autre aspect de l'invention, les compositions utilisées selon l'invention contiennent également au moins un filtre ou un écran solaire UVB et/ou UVA ; tels les écrans ou filtres minéraux et/ou organiques connus de l'homme du métier qui adaptera leur choix et leurs concentrations en fonction du degré de protection recherché.

Les compositions utilisées selon l'invention peuvent en outre contenir des agents exfoliants ou hydratants tels que les alpha-hydroxyacides et salicylique et leurs dérivés sous forme d'ester par exemple.

Les compositions utilisées selon l'invention peuvent également contenir des agents anti-inflammatoires ou apaisants, des agents désensibilisants cutanés tels que des AINS (Anti-inflammatoires non stéroïdiens), des dermocorticoïdes, des agonistes PPAR (peroxysme proliferator activated receptor : récepteur activé par les proliférateurs de peroxysomes), des dérivés de réglisse, de bisabolol, d'isoflavones (de soja par exemple) glycosilés ou non, du palmitoyléthanolamide, des insaponifiables à base de phytostérols et de vitamines E, des anti-COX et/ou LOX (inhibiteur de cyclo-oxygènase et/ou de lipoxydase), des eaux thermales, marines ou reconstituées à partir d'oligoéléments exogènes.

Enfin, les compositions utilisées selon l'invention peuvent également contenir des anti-ages : rétinol, vitamines B3, C, A ,du lycopène ou des carotènes ou tous caroténoïdes des acides alpha-hydroxy (AHA), des acides béta-hydroxy (BHA), libres, estérifiés ou greffés, des protéines de la matrice extra-cellulaire (glucosamine, chondroïtine sulfate ), du collagène, de l'élastine et leurs activateurs, des dérivés furaniques de l'avocat, des phytosterols, des acides gras insaturés et polyinsaturés, des isoflavones de plantes (soja, trèfle), des lactobacillus et autres pré et probiotiques.

Dans le cadre du traitement ou de la prévention des cheveux blancs, la composition peut en outre comprendre des activateurs de la repousse du poil et des cheveux (minoxidil, aminexil...) ou des actifs inhibant ou ralentissant leurs chutes. Les compositions peuvent aussi comprendre des agents de permanentes et colorants du cheveux, des agents filmogènes et fixant, des coiffants capillaires.

La composition utilisée selon l'invention peut être administrée par voie topique ou par voie orale.

Dans le cadre d'une application topique, la composition utilisée selon l'invention comprend un support dermatologiquement et/ou cosmétiquement acceptable, c'est à dire un support compatible avec la peau. Elle peut avantageusement se présenter sous toutes les formes galéniques normalement utilisées pour une application topique, notamment sous forme d'une solution aqueuse, hydroalcoolique ou huileuse, d'une émulsion huile-dans-eau ou eau-dans-huile ou multiple, d'un gel aqueux ou huileux, d'un produit anhydre liquide, pâteux ou solide, d'une dispersion d'huile dans une phase aqueuse à l'aide de sphérules (nanosphères, nanocapsules, vésicules lipidiques), d'un dispositif trans-dermique ou sous toute autre forme pour application topique.

Cette composition peut être plus ou moins fluide et avoir l'aspect d'une crème blanche ou colorée, d'une pommade, d'un lait, d'une lotion, d'un sérum, d'une pâte, d'une mousse ou d'un gel. Elle peut éventuellement être appliquée sur la peau sous forme d'aérosol. Elle peut également se présenter sous forme solide, et par exemple sous forme de stick. Elle peut aussi être appliquée au moyen d'un patch.

Dans le cadre d'une administration orale, la composition peut se présenter sous forme de capsule, gélule, comprimé, granule, spray ou solution buvable. Lorsque la composition se présente sous forme de capsule molle ou de gélule, l'enveloppe de ces capsules molles ou de ces gélules peut contenir notamment de la gélatine animale telle que la gélatine de poisson, de la glycérine, ou un matériau d'origine végétale tel qu'un dérivé de cellulose ou d'amidon, ou une protéine végétale. Lorsque la composition se présente sous forme de gélule, de comprimé, ou de granule, les actifs peuvent être fixés sur un support pulvérulent tel que la silice, la cellulose, et la maltodextrine.

La composition utilisée selon l'invention peut contenir également les adjuvants habituels dans le domaine cosmétique, tels que les stabilisants, les conservateurs, les antioxydants, les solvants, les parfums, les agents chélateurs, les absorbeurs d'odeur, des filtres chimiques ou minéraux, des pigments minéraux, les tensioactifs, les polymères, les huiles de silicone et les matières colorantes ou exfoliantes.

Les exemples qui suivent ont pour but l'évaluation des effets d'un actif à base de peptides de riz sur la pigmentation et la photoprotection cutanée.

Pigmentation : évaluation multiparamétrique dans les axes suivants :
- Effets sur la production de mélanine par des mélanocytes normaux humains en co-culture avec des kératinocytes (NHEM-NHEK) irradiés ou non (exemple 2)
- Effets sur l'activité de la tyrosinase dans les cultures de mélanocytes (NHEM) (exemple 3)
- Effets sur la dendricité de mélanocytes en co-culture avec des kératinocytes (NHEM-NHEK) (exemple 4)
- Effet sur le transfert de mélanosomes des mélanocytes aux kératinocytes voisins (exemple 5)
- Effets sur la pigmentation de peau en survie (exemple 6)

### Photoprotection : effet sur la synthèse du glutathion (GSH) par des fibroblastes cutanés humains irradiés aux UVA (exemple 7)

### Exemple 1 : Peptides de riz selon l'invention

Des peptides de riz sont obtenus par hydrolyse enzymatique de protéines de riz. Les protéines résiduelles sont éliminées par ultrafiltration avec un seuil de coupure à 10 000 Da. Les peptides sont concentrés par évaporation de l'eau sous vide ou par nanofiltration (200 Da) jusqu'à 5% de matière sèche. La matière sèche peut être obtenue par évaporation à sec, par lyophilisation ou par atomisation.

Les caractéristiques des peptides de riz obtenus sont résumées dans le tableau 1 suivant :

**Tableau 1 : caractéristiques des peptides de riz**

| | |
|---|---|
| Degré d'hydrolyse | 30%-40% |
| % de matière sèche (MS) | 4-6 % ou > 80 % si atomisé |
| PH | 4-7 |
| Teneur en protéines / MS | 80%-95 % |
| Teneur en azote alpha aminé / MS | 15%-25% |
| Teneur en acides aminés libres | <4% |
| Teneur en acides aminés soufrés | <3% |
| Répartition en masse molaire | |
| | > 3500 Da : <2% |
| | 3500 - 1200 Da : **20-35%** |
| | 1200 - 300 Da : **50-65%** |
| | < 300 Da : **5-15%** |

Ces peptides de riz sont ensuite testés dans les exemples 2 à 7 qui suivent.

### Exemple 2 : Dosage de la mélanine produite par des mélanocytes normaux humains en co-culture avec des kératinocytes (NHEM/NHEK), irradiés ou non.

Des co-cultures NHEM / NHEK ont été ensemencées en plaques 24 puits. Trois séries de plaques identiques ont été réalisées afin d'évaluer :
a. La quantité de mélanine par spectrophotométrie ;
b. La viabilité des cellules à la fin de l'expérience (test au MTT) ;
c. La quantité de protéines.

Après incubation 24 heures à 37°C, le milieu de culture a été éliminé et remplacé par du milieu contenant ou non (témoin) les peptides de riz (à 0,1 ; 0,5 et 2 mg/ml) ou la molécule de référence pro-pigmentante (IBMX = Isobutylmethylxanthine à 200 µM) puis les co-cultures ont été incubées à 37°C pendant 10 jours.

Un lot de plaques n'a pas été irradié (lot co-culture NHEM / NHEK non irradiée).

Le second lot a été irradié : 4 jours consécutifs par une dose de 25 mJ/cm² d'UVB + 300 mJ/cm² d'UVA puis le milieu de culture contenant ou non les produits (peptides de riz ou IBMX) a été renouvelé, les co-cultures ont été cultivées 72 h sans irradiation puis à nouveau irradiées à 25 mJ/cm² UVB + 300 mJ/cm² d'UVA pendant 4 jours consécutifs (avec changement du milieu au bout de 48 h).

A la fin du traitement, les milieux de culture ont été éliminés et la mélanine contenue dans les cellules a été extraite par une solution de NaOH 0,5 N, puis la quantité de mélanine produite dans chaque condition de co-culture a été évaluée par mesure de la densité optique des échantillons à 405 nm contre une gamme de mélanine exogène.

### Viabilité cellulaire et quantité de protéines

Les résultats du test au MTT sont présentés dans le tableau 2 suivant :

**Tableau 2 : Evaluation de la viabilité cellulaire dans les co-cultures NHEM/NHEK**

| Echantillon | % de viabilité dans les co-cultures non irradiées | % de viabilité dans les co-cultures irradiées |
|---|---|---|
| Témoin | 100 | 100 |
| IBMX à 200 µM | 118 | 111 |
| Peptides de riz à 0,1 mg/ml | 122 | 112 |
| Peptides de riz à 0,5 mg/ml | 131 | 122 |
| Peptides de riz à 2,0 mg/ml | 138 | 126 |

Les résultats du dosage des protéines sont présentés dans le tableau 3 suivant :

**Tableau 3 : Dosage des protéines totales dans les co-cultures**

| Echantillon | Protéines (mg/ml) dans les co-cultures non irradiées | Protéines (mg/ml) dans les co-cultures irradiées |
|---|---|---|
| Témoin | 0,828 | 0,767 |
| IBMX à 200 µM | 0,933 | 0,833 |
| Peptides de riz à 0,1 mg/ml | 0,864 | 0,742 |
| Peptides de riz à 0,5 mg/ml | 0,890 | 0,774 |
| Peptides de riz à 2,0 mg/ml | 0,942 | 0,973 |

Les peptides de riz ne présentent aucune toxicité sur les co-cultures NHEM / NHEK (même remarque pour l'IBMX). Au contraire, une augmentation de la quantité de protéines et de l'activité métabolique des cellules en présence des peptides de riz comme de l'IBMX a été mise en évidence.

### Dosage de la mélanine

Les résultats sont présentés dans le tableau 4 suivant :

**Tableau 4 : Modulation de la production de la mélanine dans les co-cultures NHEM/NHEK**

| Echantillon | % d'augmentation de la quantité de mélanine par rapport au témoin, dans les co-cultures non-irradiées | % d'augmentation de la quantité de mélanine par rapport au témoin, dans les co-cultures irradiées |
|---|---|---|
| IBMX à 200 µM | +18 % * | +14 % * |
| Peptides de riz à 0,1 mg/ml | +15 % * | +7 % $ |
| Peptides de riz à 0,5 mg/ml | +12 % * | +5 % |
| Peptides de riz à 2,0 mg/ml | +29 % * | +11 % * |

| | | |
|---|---|---|
| * Statistiquement différent du témoin (p<0,01, test Dunnett) $ Statistiquement différent du témoin (p<0,05 ; test Dunnett) | | |

Dans les conditions non irradiées, les peptides de riz testés à 0,1, 0,5 et 2,0 mg/ml ont significativement augmenté la quantité de mélanine (respectivement +15 %, +12 % et +29 % par rapport au témoin, p<0,01).

Dans les conditions irradiées, les peptides de riz testés à 2 mg/ml ont significativement augmenté la quantité de mélanine (+11 % par rapport au témoin, p<0,01). A 0,5 mg/ml et 0,1 mg/ml, cette stimulation était moins marquée.

Les peptides de riz agissent sur la synthèse de mélanine de la même manière que la molécule de référence pro-pigmentante (IBMX).

De plus, comme pour l'IBMX, la stimulation de la synthèse de mélanine par les peptides de riz est à corréler avec une augmentation de la quantité de protéines et de l'activité métabolique des cellules (% viabilité) (cf. tableaux 2 et 3).

### Exemple 3 Effets sur l'activité de la tyrosinase dans des cultures de mélanocytes (NHEM).

Les mélanocytes (NHEM) ont été pré-cultivés en plaques 96 puits pendant 24 heures, puis les cellules ont été traitées par les peptides de riz ou l'IBMX (200µM), pendant 72 heures à 37°C.

A la fin du traitement, la tyrosinase contenue dans les cellules a été extraite, la réaction d'oxydation de la L-DOPA en DOPAquinone a été suivie après ajout de la L-DOPA (substrat de la réaction) pendant 1 heure à 37°C. L'activité tyrosinase a ensuite été évaluée par mesure de la densité optique des échantillons à 450 nm, contre une gamme étalon de tyrosinase.

Les résultats sont présentés dans le tableau 5 suivant :

**Tableau 5: Effets des traitements sur l'activité tyrosinase de mélanocytes humains**

| Echantillon | Activité tyrosinase (unité/ml) | % d'augmentation par rapport au témoin |
|---|---|---|
| Témoin | 29,12 | - |
| IBMX à 200 µM | 60,88 * | +109 % |
| Peptides de riz à 0,5 mg/ml | 46,29 * | +59 % |
| Peptides de riz à 2,0 mg/ml | 79,18 * | +172 % |

| | | |
|---|---|---|
| * Statistiquement différent du témoin (p<0,01, test Dunnett) | | |

Les peptides de riz, testés à 0,5 mg/ml et 2 mg/ml, ont significativement stimulé l'activité tyrosinase de mélanocytes humains, suivant un effet dose-dépendant (respectivement +59% et +172% par rapport au témoin, p<0,01).

### Exemple 4: Evaluation de la dendricité de mélanocytes en co-culture avec des kératinocytes (NHEM / NHEK)

Les co-cultures NHEM / NHEK ont été ensemencées 24 heures avant le test en plaques 96 puits, puis ont été incubées pendant 72 heures en présence ou non (témoin) des peptides de riz (à 0,1 ; 0,5 et 2,0 mg/ml) ou de l'IBMX (à 200 µM).

La dendricité a été mise en évidence par un marquage fluorescent à l'aide d'un anticorps anti-MEL5 (ou TRP1 - Tyrosinase Related Protein 1) spécifique des mélanosomes). Le marquage a été révélé par un anticorps secondaire GAM-FITC.

Cette analyse a permis de déterminer la densité des prolongements des mélanocytes (nombre et longueur totale des dendrites par mélanocytes).

### Effet sur la dendricité de mélanocytes en co-culture avec des kératinocytes

**Tableau 6 : Mesure de la dendricité des mélanocytes dans des co-cultures NHEM / NHEK : Analyse du nombre de dendrites par mélanocytes.**

| Traitement | Nombre moyen de dendrites par mélanocyte | % d'augmentation par rapport au témoin par rapport au témoin |
|---|---|---|
| Témoin | 12,18 | - |
| IBMX à 200 µM | 15,30 * | + 26 % |
| Peptides de riz à 0,1 mg/ml | 12,73 | + 4 % |
| Peptides de riz à 0,5 mg/ml | 14,52 * | + 19 % |
| Peptides de riz à 2,0 mg/ml | 15,43 * | + 27% |

| | | |
|---|---|---|
| * Statistiquement différent du témoin (p<0,01, test Dunnett) | | |

**Tableau 7 : Mesure de la dendricité des mélanocytes dans des co-cultures NHEM / NHEK : Analyse de la longueur des dendrites.**

| Traitement | Longueur moyenne des dendrites par mélanocyte | % d'augmentation par rapport au témoin |
|---|---|---|
| Témoin | 228,18 | - |
| IBMX à 200 µM | 402,50 $ | + 76 % |
| Peptides de riz à 0,1 mg/ml | 276,09 | + 21% |
| Peptides de riz à 0,5 mg/ml | 285,20 | + 25 % |
| Peptides de riz à 2,0 mg/ml | 381,82 * | + 67 % |

| | | |
|---|---|---|
| *$ Statistiquement différent du témoin (* p<0,01, $ p<0,05, test Dunnett) | | |

Les peptides de riz testés à 2 mg/ml et 0,5 mg/ml ont augmenté de façon dose-dépendante le nombre et la longueur des prolongements des mélanocytes. A 0,1 mg/ml, l'effet sur le nombre de dendrites n'est plus visible par contre on observe encore un léger effet sur la longueur.

### Exemple 5 : Effet sur le transfert de mélanosomes des mélanocytes aux kératinocytes voisins.

### Protocole:

Avant ensemencement, les mélanocytes ont été « chargés » avec la sonde fluorescente traceuse du transfert de mélanosomes « succinimidyl ester of carboxy fluorescein diacetate » (CFDA, en français : diacétate de carboxyfluorescéine, succinimidyl ester).

Les mélanocytes « chargés » ont ensuite été incubés en co-culture avec les kératinocytes à 37°C (NHEM/NHEK). Plusieurs contrôles ont été réalisés afin de procéder aux réglages des paramètres d'acquisition par cytometrie de flux :
- Co-culture NHEM / NHEK « non chargés ».
- Cultures NHEK seuls.
- Cultures NHEM « chargés » ou non, testés seuls.

A 60-80% de confluence, le milieu de culture a été remplacé par du milieu contenant ou non (témoin) les peptides de riz (0,5 et 2 mg/ml) ou l'IBMX, puis les cellules ont été cultivées à 37°C. Après 72h d'incubation, le traitement des cellules a été renouvelé comme précédemment et les cellules ont été à nouveau incubées 72h à 37°C. Chaque condition de traitement a été réalisée en triplicata.

A la fin du traitement, les cellules ont été trypsinées, fixées, perméabilisées, puis marquées avec un anticorps anti-cytokératines (spécifique des kératinocytes). Le marquage a été révélé à l'aide d'un anticorps secondaire dirigé contre l'anticorps anti-cytokératines et couplé à la phycoérythrine. Les paramètres de fluorescence ont été mesurés par cytometrie de flux.

### Analyse des résultats par cytometrie de flux:

L'analyse cytométrique a été paramétrée (grâce aux différents contrôles réalisés) afin de sélectionner dans la population cellulaire totale (NHEM/NHEK) uniquement les kératinocytes positifs ; c'est à dire ayant incorporé la sonde fluorescente CFDA (indiquant le transfert des mélanosomes) et présentant un marquage anti-cytokératines (spécifique des kératinocytes). Cette population de kératinocytes «doublement marquée » est obligatoirement le résultat d'un transfert de mélanosomes du mélanocyte vers les kératinocytes.

### Résultats et conclusions:

Les résultats sont présentés dans le tableau 8 suivant :

**Tableau 8 : effet des traitements sur le transfert de melanosomes des mélanocytes aux kératinocytes**

| Echantillon | % moyen de cellules positives (double marquage CFDA/Cytokératines) | % d'augmentation par rapport au témoin |
|---|---|---|
| Témoin | 18,1 | - |
| IBMX à 200 µM | 26,7 * | + 48 % |
| Peptides de riz à 0,5 mg/ml | 23,3 * | +29 % |
| Peptides de riz à 2 mg/ml | 23,1 ^{$} | +28 % |

| | | |
|---|---|---|
| *$ Statistiquement différent du témoin (* p<0,01, $ p<0,05, test Dunnett) | | |

Les peptides de riz testés à 0,5 mg/ml et 2 mg/ml ont induit une augmentation significative du transfert de mélanosomes (respectivement 29% et 28% par rapport au témoin). Les peptides de riz présentent donc un effet stimulant sur le transfert des mélanosomes.

### Exemple 6 : Effets sur la pigmentation de peau en survie

Des échantillons de peau obtenus à partir d'une biopsie mammaire ont été traités par application topique des peptides de riz (à 0,1 ; 0,5 et 2,0 mg/ml) ou de l'IBMX (à 200 µM) (dépôt de 20 µl, soit 5 mg/cm² environ). Les explants ont ensuite été maintenus pendant 144 heures à 37°C.

Les zones traitées et contrôles ont été photographiées à la fin du traitement et la coloration de la peau a été analysée visuellement.

L'IBMX a légèrement augmenté la pigmentation de la peau après 144 heures de culture.

Les peptides de riz ont augmenté de façon dose dépendante la pigmentation de la peau après 144h de culture. L'actif présente un effet pro-pigmentant en accord avec les résultats obtenus dans les autres tests réalisés.

### CONCLUSION DES EXEMPLES 2 à 6 :

Les exemples précédemment cités ont permis de mettre en évidence un effet pro-pigmentant des peptides de riz. En effet, il a été démontré que les peptides de riz augmentent la synthèse de mélanine dans des co-cultures NHEM / NHEK irradiées ou non ; augmentent l'activité tyrosinase (enzyme clé dans la mélanogénèse) ; augmentent le nombre et la longueur des prolongements des mélanocytes ; augmentent le transfert de mélanosomes des mélanocytes aux kératinocytes et augmentent la pigmentation d'explants de peau.
De plus l'activité des peptides de riz, dans les différentes études réalisées, est comparable à l'activité de l'IBMX, utilisé ici comme molécule de référence pro-pigmentante.

L'actif à base de peptides de riz est donc un accélérateur de bronzage. En effet, avec ou sans exposition aux UV, il induit une forte stimulation de la pigmentation cutanée.

### Exempte 7 : effet sur la synthèse du glutathion (GSH) par des fibroblastes cutanés humains irradiés aux UVA

Les fibroblastes dermiques ont été ensemencés en boîtes de Pétri d'un diamètre de 60 mm. Les cellules ont été pré-traitées pendant 18 heures par les peptides de riz ou la N-Acétyl Cystéine à 5 mM (NAC = molécule anti-oxydante de référence), puis irradiées par une dose de 15 J/cm² d'UVA. Après irradiation, les fibroblastes ont été incubés 3 heures supplémentaires à 37°C.

A la fin du traitement, deux séries d'échantillons ont été récoltées afin d'évaluer :
a. La quantité de protéines totales selon la méthode de Lowry
b. Le taux de glutathion intracellulaire par dosage (kit commercialisé)

Les résultats sont exprimés dans le tableau 9 suivant :

**Tableau 9 : modulation du glutathion intracellulaire de fibroblastes dermiques irradiés aux UVA.**

| | GSH (µM) | Protéines totales (g/l) | GSH (µmol/g protéines) | % d'augmentation/cellules contrôles irradiées) |
|---|---|---|---|---|
| Cellules contrôles | 18,30 | 0,764 | 23,95 | - |
| Cellules contrôles +UVA 15J/cm² | 15,50 | 0,708 | 21,89 | -8,60% vs cellules contrôles |
| NAC (5 mM) | 21,10 | 0,748 | 28,209 | 29 |
| Peptides de riz 0,5% MS | 17,70 | 0,718 | 24,65 | 13 |
| Peptides de riz 1% MS | 18,70 | 0,714 | 26,19 | 20 |
| Peptides de riz 1,5% MS | 21,20 | 0,753 | 28,15 | 29 |

Une irradiation à la dose de 15 J/cm² fait chuter de presque 9% le taux de GSH intracellulaire des fibroblastes par rapport aux cellules non irradiées.

La NAC, à la dose de 5 mM, augmente de 29% le contenu en GSH des cellules irradiées.

Les peptides de riz aux concentrations de 0,5-1 et 1,5% MS augmentent de respectivement 13, 20 et 29% le contenu en GSH de fibroblastes soumis à un stress UVA.

En d'autres termes, les peptides de riz sont capables de compenser la diminution des taux intracellulaires de GSH suite à une irradiation UVA.

### CONCLUSION GENERALE :

Les peptides de riz permettent de stimuler le bronzage tout en protégeant la peau des effets nocifs du soleil.

### Exemple 8 : formulations

### 8a - crème visage

| | % |
|---|---|
| Isononyl Isononanoate | 6,000 |
| Di-C₁₂₋₁₃ Alkyl Malate | 3,000 |
| Stéarate isocétyle | 4,000 |
| Butylène glycol | 1,000 |
| **Peptides de riz** | **3,000** |
| Dicaprylyl Ether | 5,000 |
| Salicylate de Silanediol | 1,000 |
| Alcool arachique | 1,650 |
| Trométhamine | 1,180 |
| Alcool cétylique | 0,5 |
| Acide salicylique | 1,000 |
| Glucoside ascorbyl | 1,000 |
| Glycine | 1,000 |
| Acétate de tocopheryl | 1,000 |
| Alcool béhénylique | 0,900 |
| Squalane | 0,790 |
| Citrate de Sodium | 0,660 |
| Copolymère PPG-12/SMDI | 0,100 |
| Arachidyl Glucoside | 0, 350 |
| Parfum | 0,400 |
| Gomme sclerotium | 0,300 |
| Alcool cétéarylique | 0,430 |
| Acide citrique | 0,110 |
| Sepigel 305* | 0,100 |
| Système conservateur | QS |
| Eau | QSP 100 |

| | |
|---|---|
| *produit commercialisé par la société Seppic | |

### 8b - Cosmétique anti age solaire et bronzante (Quantité : 100g)

| | % |
|---|---|
| Eau | 64,83 |
| **Peptides de de riz** | **7** |
| **Insaponifiable d'avocat furanique** | **2** |
| Butylène glycol | 3 |
| Alcool cétylique | 6 |
| Alkyl lactate C12-13 | 2 |
| Huile minérale | 7 |
| Ceteth 20 | 2 |
| Acide stéarique | 1 |
| Cinnamate | 5 |
| Mexoryl SX | 3 |
| Mexoryl XL | 4 |
| Dioxyde de titane ultrafin | 7 |
| Oxyde de zinc | 2 |
| Tinosorb M et S | 4 |
| Acétate de Tocophérol | 0,50 |
| Proplène glycol | 0,56 |
| EDTA dissodique | 0,52 |
| Triéthano lamine | 0,80 |
| Conservateur | 0,30 |
| Acide citrique | 0,25 |
| Methyl paraben | 0,11 |
| Steareth -20 | 1 |
| Metabisulfite de sodium | 0,05 |
| Sulfite de sodium | 0,05 |
| Propyl paraben | 0,03 |

### 8c - rollon anti tâche blanche (Quantité : 100g)

| | % |
|---|---|
| Vaseline officinale | 6,00 |
| Huile palmiste hydrogénée | 4,00 |
| Caprylo caprate de glycérol | 2,00 |
| Sucro ester 7 (Distérate sucrose)) | 6,00 |
| Squalane | 1,00 |
| Cire de candelilla | 2,00 |
| Sucro ester 11 (Stéarate sucrose) | 0,50 |
| **Peptides de riz** | **2,00** |
| Concentrat de tournesol | 2,00 |
| Glycérol | 5,00 |
| G lucodextrine | 1,00 |
| Trométamine | 0,01 |
| Gomme xanthane | 0,20 |
| Hydroxyméthylglycinate A | 0,60 |
| Acide citrique | 0,32 |
| Cyclométhiconol | 5,00 |
| Céramide/Cholestérol | 0,60 |
| Eau purifiée | 5,00 |

### 8d - crème d'entretien du hâle d'été (Quantité : 100g)

| | % |
|---|---|
| Squalane | 1,00 |
| Erythrityl ester | 4,00 |
| Decyl pentanoate | 4,00 |
| Cetearyl glucoside | 2,00 |
| Lauryl ether 23 OE | 1,00 |
| Cutina CBSV | 1,00 |
| Cire d'abeille | 0,50 |
| Myristate myristyle | 1,00 |
| Conservateur | 0,30 |
| Vaseline épaisse | 5,00 |
| Squalane gelifiée | 3,00 |
| **Peptides de riz** | **5,00** |
| Eau purifiée | 56,51 |
| Phenoxyéthanol | 0,80 |
| EDTA de sodium | 0,10 |
| Acide citrique | 0,14 |
| Sorbate de potassium | 0,45 |
| Glycérine | 5,00 |
| Epaississant | 0,50 |
| Lessive de soude | 0,30 |
| Polyacrylamide gel 60° | 1,00 |
| Acétate vitamine E 35° | 0,50 |
| Parfum | 0,50 |
| Peptide de lupin | 1,00 |
| Cyclométhyconol | 7,00 |
| Silice TIO2 | 1,00 |
| Génisteine 85% | 0,10 |
| PEG 300 | 0,90 |

### 8e - Crème bronzante pour le vitiligo et les hypopigmentations (Quantité : 100g)

| | % |
|---|---|
| Montanov 68 | 3 |
| Amphisol K | 0,50 |
| Miglyol 812 | 6 |
| Conservateur | 0,30 |
| Beurre de karité | 1 |
| **Peptides de riz** | **1** |
| **polyphénol** | **0,5** |
| **DHA** | **2** |
| EDTA Na₂ | 0,10 |
| Acide citrique | 0,01 |
| Conservateur | 0,40 |
| Butylène glycol | 1 |
| Gélifiant | 0,25 |
| Lessive de soude | 0,4 |
| Gluconate manganèse | 0,05 |
| Zinc de sel | 0,10 |
| Eau purifiée | QSP |

### 8f- Capsule molle nutraceutique capillaire autobronzant et fortifiant

| | % |
|---|---|
| Cystéine ou dérivés acides aminés soufrés | 0,100 |
| **Peptides de riz** | **500** |
| Vitamine du groupe B | 1 |
| Hydrolysats de pois | 50 |
| Hydrolysats de plume de canard | 50 |
| Excipient | QS |

### 8g - Traitement des hypopigmentations par voie orale

| | % |
|---|---|
| Mucilage (alginate de sodium) | 500 mg |
| Huile de poisson | 500 mg |
| **Peptides de riz** | **100 mg** |
| Sel de zinc | 1 mg |
| Extrait de carotte | 325 mg |
| lycopène | 50 mg |
| Excipient capsule molle | QS |

### 8h - Gel crème Antiage et bronzant sans soleil (Quantité : 100g)

| | % |
|---|---|
| Carbopol Etd 2020 | 0,6 |
| Gomme xanthane | 0,15 |
| Génisteine 85% | 0,1 |
| Rétinol | 0,5 |
| NaOH | 0,001 |
| Conservateur | 0,9 |
| **Peptides de riz** | **2** |
| G lucodextrine | 2 |
| Parfum | 0,7 |
| Silicone | 0,3 |
| Eau purifiée | QSP |

### 8i - Lingettes pigmentantes (Quantité : 100g)

| | % |
|---|---|
| Poloxamer 184 | 1,0000 |
| Parfum | 0,2000 |
| Eau purifiée | 91,1550 |
| PEG - 32 | 4,0000 |
| Conservateur | 1,0000 |
| Chlorhexidine | 0,1500 |
| Phenoxyéthanol | 0,1000 |
| Allantoine | 0,2000 |
| **peptides de riz** | **5,0000** |
| Solubilisant | 1,0000 |
| Trométhamine | 0,1950 |

### 8j - Solution hydro glyco alcoolique pour la repousse et la coloration des cheveux

| | % |
|---|---|
| Alcool dénaturé | 70% |
| Cyclodextrine | 2% |
| Glycol | 25 % |
| Aminexil | 1% |
| Minoxidil | 0,3% |
| Eau | qsp 100% |

## Revendications

1. Utilisation non thérapeutique d'un hydrolysat de protéines de riz pour pigmenter, hyperpigmenter ou repigmenter la peau et les phanères.

2. Utilisation selon la revendication 1 pour des applications choisies dans le groupe constitué par l'augmentation et l'intensification de la pigmentation normale de la peau sans soleil, l'accélération et l'intensification du bronzage, et la prévention du photovieillissement.

3. Utilisation selon la revendication 1 destinée à repigmenter les tâches blanches cutanées, en particulier les tâches consécutives à une affection ou une sensibilisation choisie dans le groupe constitué par un vitiligo, un pityriasis, une utilisation de dermo-corticoïdes une cicatrice ou une exposition solaire intensive.

4. Utilisation selon la revendication 1 destinée au soin capillaire pour la prévention et le traitement des cheveux blancs.

5. Utilisation selon l'une quelconque des revendications précédentes, **caractérisée en ce que** l'hydrolysat de protéines de riz comprend des peptides dont au moins 50% ont une masse moléculaire comprise entre300 et 10 000 Da, plus particulièrement entre 300 et 3 500 Da.

6. Utilisation selon la revendication 5, **caractérisée en ce qu'**au moins 50% des peptides ont une masse moléculaire comprise entre 300 et 1 200 Da.

7. Utilisation selon l'une quelconque des revendications précédentes, **caractérisée en ce que** l'hydrolysat de protéines de riz est present dans une composition en concentration allant de 0,1 à 20% en poids, par rapport au poids total de la composition.

8. Utilisation selon l'une quelconque des revendications précédentes, **caractérisée en ce que** on utilise en outre des agents anti-oxydants, photo-protecteurs, hydratants, colorants, hyper-pigmentants, anti-âges.

9. Méthode de traitement cosmétique non thérapeutique pour stimuler la pigmentation constitutive ou acquise, **caractérisée en ce que** l'on applique par voie topique une composition comprenant au moins un hydrolysat de protéines de riz.

10. Méthode de traitement cosmétique selon la revendication 9, destinée à accélérer le bronzage et la couleur naturelle de la peau sans soleil.

## Claims

1. A non therapeutic use of a rice protein hydrolysate to pigment, hyper-pigment or re-pigment the skin and skin structures.

2. The use according to claim 1, for applications chosen among the group comprised of increasing and intensifying normal pigmentation of the skin without sunlight, accelerating and intensifying tanning, and preventing photoageing.

3. The use according to claim 1, intended for re-pigmenting cutaneous white spots, in particular spots consecutive to an affection or sensitization chosen among the group comprised of vitiligo, pityriasis, use of dermal corticosteroids, scarring or intense exposure to the sun.

4. The use according to claim 1, intended for a capillary treatment to prevent and to treat white hair.

5. The use according to any of the preceding claims, **characterized in that** the rice protein hydrolysate comprises peptides of which at least 50% have a molecular weight between 300 and 10,000 Da, more particularly between 300 and 3,500 Da.

6. The use according to claim 5, **characterized in that** at least 50% of the peptides have a molecular weight between 300 and 1,200 Da.

7. The use according to any of the preceding claims, **characterized in that** the rice protein hydrolysate is present in a composition in a concentration between 0.1% to 20% by weight, compared to the total weight of the composition.

8. The use according to the preceding claim, **characterized in that** one further use antioxidants, photoprotectors, hydrating agents, dyes, hyper-pigmenting agents or anti-ageing agents.

9. A method of non therapeutic cosmetic treatment to stimulate constitutive or acquired pigmentation, **characterized in that** a composition comprising at least one rice protein hydrolysate is applied by topical route.

10. The method of cosmetic treatment according to claim 9, intended to accelerate the tanning and the natural color of the skin without sunlight.

## Patentansprüche

1. Nicht-therapeutische Verwendung eines Hydrolysats von Reisproteinen, um die Haut und die Haare und Nägel zu pigmentieren, hyperpigmentieren oder repigmentieren.

2. Verwendung nach Anspruch 1, für Anwendungen, die ausgewählt sind aus der Gruppe bestehend aus der Verstärkung und Intensivierung der normalen Pigmentierung der Haut ohne Sonne, der Beschleunigung und der Intensivierung des Bräunens und der Verhütung von Lichtalterung.

3. Verwendung nach Anspruch 1, dazu bestimmt, um weiße Hautflecken zu repigmentieren, insbesondere Flecken als Folge einer Krankheit oder Sensibilisierung, welche ausgewählt ist aus der Gruppe bestehend aus Vitiligo, Pityriasis, einer Verwendung von Hautkortikoiden, einer Narbe und/oder einer intensiven Sonneneinwirkung.

4. Verwendung nach Anspruch 1, die zur Haarpflege für die Verhütung oder Behandlung von weißen Haaren bestimmt ist.

5. Verwendung nach irgendeinem der vorangehenden Ansprüche, **dadurch gekennzeichnet, dass** das Hydrolysat von Reisproteinen Peptide umfasst, von denen mindestens 50 % eine Molekularmasse zwischen 300 und 10000 Da, spezieller zwischen 300 und 3500 Da aufweisen.

6. Verwendung nach Anspruch 5, **dadurch gekennzeichnet, dass** mindestens 50 % der Peptide eine Molekularmasse zwischen 300 und 1200 Da einschließlich aufweisen.

7. Verwendung nach irgendeinem der vorangehenden Ansprüche, **dadurch gekennzeichnet, dass** das Hydrolysat von Reisproteinen in einer Zusammensetzung mit einer Konzentration von 0,1 bis 20 Gew.-%, bezogen auf das Gesamtgewicht der Zusammensetzung vorliegt.

8. Verwendung nach dem vorangehenden Anspruch, **dadurch gekennzeichnet, dass** man darüber Antioxidantien, Lichtschutzmittel, Befeuchtungsmittel, Färbemittel, Hyperpigmentierungsmittel, Antialterungsmittel verwendet.

9. Verfahren zur kosmetischen, nicht-therapeutischen Behandlung zur Stimulierung der konstitutiven oder erworbenen Pigmentierung, **dadurch gekennzeichnet, dass** man auf topischem Weg eine Zusammensetzung aufträgt, die mindestens ein Hydrolysat von Reisproteinen umfasst.

10. Verfahren zur kosmetischen Behandlung nach Anspruch 9, das dazu bestimmt ist, das Bräunen und die natürliche Farbe der Haut ohne Sonne zu beschleunigen.
